# FASCICULE DE BREVET EUROPEEN

(11) **EP 1 532 930 B1**
(45) Date de publication et mention de la délivrance du brevet: **29.11.2006**
(21) Numéro de dépôt: 04029007.4
(22) Date de dépôt: 21.04.1999
(51) Int. Cl.: A61B 17/04, F16B 13/06

(54) **Ancre de suture à expansion réversible**
Wiederlösbarer Spreizanker für chirurgischen Nähfaden
Suture anchor with reversible expansion

(30) Priorité: 21.04.1998 FR 9805203
(43) Date de publication de la demande: 25.05.2005
(62) Demande divisionnaire de: 99914634.3
(73) Titulaire: TORNIER, 38330 Saint-Ismier (FR)
(72) Inventeur: Tornier, Alain, 38330 Saint Ismier (FR); Bonnomet, Francois, 67000 Strasbourg (FR)
(74) Mandataire: Myon, Gérard Jean-Pierre

(56) Documents cités:
- WO-A-90/00370
- GB-A- 2 173 565
- US-A- 5 472 452
- US-A- 5 501 695
- US-A- 5 649 963

## Description

La présente invention est relative à une ancre de suture permettant de rattacher des tissus mous tels que des ligaments ou tendons à l'os, et principalement pour la réparation de la coiffe des rotateurs et des lésions de Bankart.

On connaît d'après le document U.S. 5 501 695 au nom de Anspach, sur lequel est basée la forme en deux parties de la revendication 1, une ancre de suture en deux parties permettant la réinsertion musculaire et tendineuse sur l'os.

Cette ancre de suture comprend un premier élément extérieur cylindrique solidaire de branches de fixation qui sont séparées les unes des autres par des fentes disposées parallèlement à l'axe longitudinal de l'ancre de suture. Le premier élément reçoit dans sa partie interne un second élément de déformation qui coopère avec l'extrémité libre des branches de fixation.

Le second élément de déformation est solidaire par l'intermédiaire d'une zone de rupture d'une tige de traction qui permet au chirurgien, après avoir introduit l'ancre de suture dans un trou préalablement ménagé dans l'os, de faire coulisser ledit second élément à l'intérieur du premier afin de déformer axialement les branches de fixation dans la partie de l'os spongieux.

Lorsque l'effort de traction est suffisant pour déformer les branches de fixation la tige se sépare du second élément par une rupture irréversible.

On note que les branches se déforment latéralement suivant une direction sensiblement perpendiculaire à l'axe longitudinal de l'ancre de suture pour fixer définitivement cette dernière à l'intérieur de l'os.

Enfin, le premier élément est solidaire à l'une de ses extrémités d'une collerette qui vient en appui contre l'os cortical et qui est percée d'un certain nombre de trous pour la fixation par le chirurgien de fils de suture.

L'ancre de suture décrite ci-dessus comporte certains inconvénients à savoir qu'elle n'est pas prévue pour être retirée de l'os sans provoquer une destruction complète de cette dernière et de l'os dans laquelle elle est fixée. En effet l'ancre de suture ne comporte aucun moyen de reprise permettant son retrait de l'os sans engager une détérioration de ce dernier.

En outre, l'ancre de suture ne comporte pas en dehors de la zone de rupture du second élément, des moyens limitant la course dudit élément pour éviter que les branches de fixation viennent à se rompre anormalement sous l'effort de traction.

On connaît également du brevet US 5 649 963, une ancre de suture et un outil sur lequel se visse une douille pour la fixation de ladite ancre.

L'ancre de suture comporte des moyens d'expansion qui s'écarte en direction de l'extérieur sous la pression de la douille et de l'outil. On note que l'outil présente un dispositif qui permet de rapprocher les moyens d'expansion pour l'extraction de l'ancre de suture.

On constate que l'ancre de suture ne comporte pas de moyens de butée limitant la déformation des moyens d'expansion lors de l'application de la pression de la douille et de l'outil.

On connaît d'après le document US 5 472 452, une ancre de suture comportant des moyens d'expansion à déformation élastique, et un dispositif de déformation guidé à l'intérieur du corps creux de ladite ancre pour déformer les moyens d'expansion.

Le dispositif de déformation présente un profil particulier permettant sous un effort de traction de déformer les moyens d'expansion élastique afin qu'ils pénètrent à l'intérieur de l'os spongieux qui se trouve au voisinage du trou dans lequel est préalablement introduit l'ancre de suture.

Le dispositif de déformation présente un profil particulier permettant aux moyens d'expansion d'être bloqués dans une position qui retient l'ancre de suture dans l'os spongieux.

Lors d'un effort de poussée, le dispositif de déformation pénètre à l'intérieur du corps de l'ancre de suture pour déformer à nouveau les moyens d'expansion dans une direction identique à celle de l'effort de traction.

On constate que lors du retrait de l'ancre de suture, les moyens d'expansion pénètrent plus profondément à l'intérieur de l'os spongieux, risquant d'empêcher le retour des moyens d'expansion dans leur position d'origine qui est obtenue du fait des caractéristiques élastiques desdits moyens d'expansion.

On connaît d'après le document GB 2 173 565 une vis chirurgicale constituée de plusieurs pièces coopérant intimement ensemble pour permettre la déformation de moyens d'expansion réalisés dans une matière plastique à haute densité.

La vis chirurgicale comporte un corps creux et cylindrique traversé par un élément fileté pourvu d'une tête, d'un écrou de serrage coopérant avec l'élément fileté, et des moyens d'expansion prévus entre le corps cylindrique et la tête de l'écrou de serrage.

Lors de l'entraînement en rotation de l'élément fileté, l'écrou pénètre à l'intérieur du corps creux afin de déformer les moyens d'expansion qui sont collés à la tête dudit écrou.

On note que la vis décrite dans le brevet GB 2 173 565 n'est pas monobloc, c'est à dire réalisée d'une seule pièce, et que les moyens d'expansion ne présentent pas de caractéristiques de déformation plastique du fait de la matière utilisée.

C'est à ces inconvénients qu'entend plus particulièrement remédier la présente invention.

L'ancre de suture suivant la présente invention à pour objet d'être réversible pour permettre son extraction de l'os sans avoir à percer un trou à un diamètre plus grand que celui des branches déformées.

L'ancre de suture suivant la présente invention permettant la fixation de tissus mous contre un os, comporte un corps creux comprenant à l'une de ses extrémités une tête pourvue de moyens de fixation des tissus mous contre l'os par le passage d'au moins un fil de suture, lesdits moyens de fixation sont constitués de deux languettes opposées et parallèles à l'axe longitudinal (XX') du corps creux, avant déformation, afin de délimiter un espace oblong qui est traversé par au moins un fil de suture, ledit fil de suture étant serti sur la tête après déformation des languettes sous un effort de traction pour permettre la ligature des tissus mous sur l'ancre de suture.

L'ancre de suture suivant la présente invention comporte un corps creux comprenant des moyens d'expansion réversibles qui sont constitués par au moins deux branches de fixation disposées dans le prolongement de ladite tête et parallèlement à l'axe longitudinal (XX') dudit corps avant déformation, au moins deux butées intercalées entre chaque branche de fixation et limitant la déformation plastique de ces dernières lors de l'application d'un effort extérieur de traction (T) pour la fixation de l'ancre de suture dans l'os afin que la déformation desdits moyens d'expansion soit réversible lors de l'application d'un autre effort extérieur de poussée (P) permettant le retrait de ladite ancre de suture de l'os.

L'ancre de suture suivant la présente invention comporte un corps creux comprenant une pointe à profil conique dans laquelle est ménagée un trou borgne fileté qui est prévu pour recevoir une tige filetée d'un ancillaire pour déformer, d'une part sous un premier effort de traction (T) les branches de fixation suivant une direction sensiblement perpendiculaire à l'axe longitudinal (XX') dudit corps et d'autre part sous un second effort de traction (T1, T2) supérieur au premier (T) les moyens de fixation.

L'ancre de suture suivant la présente invention comporte un corps creux comprenant à proximité de la tête un alésage interne fileté qui est destiné à recevoir une première tige filetée creuse d'un ancillaire, tandis qu'une seconde tige coulissant dans la première tige vient en appui dans le fond d'un trou borgne fileté pour déplier sous un effort de poussée (P) les branches de fixation dans une position sensiblement allongée pour pouvoir extraire l'ancre de suture de l'os.

L'ancre de suture suivant la présente invention comporte des branches de fixation qui sont raccordées à une partie cylindrique de la tête et à la pointe à profil conique par des premières amorces de pliage dirigées en direction du centre du corps.

L'ancre de suture suivant la présente invention comporte des branches de fixation qui présentent respectivement en leur milieu une seconde amorce de pliage qui est inversée par rapport aux premières amorces de pliage de manière que chaque branche soit constituée de deux segments identiques.

L'ancre de suture suivant la présente invention comporte un alésage interne fileté et un trou borgne fileté qui sont portés par le même axe longitudinal (XX') du corps et sont prévus de diamètres différents.

L'ancre de suture suivant la présente invention comporte des butées qui s'étendent parallèlement à l'axe longitudinal (XX') du corps et présentent une longueur qui dépend de la déformation que l'on désire obtenir des branches de fixation.

L'ancre de suture suivant la présente invention comporte des moyens de fixation dont l'espace oblong est positionné de manière que sa direction de plus grande longueur soit perpendiculaire à l'axe longitudinal (XX') du corps.

L'ancre de suture suivant la présente invention comporte une tête comprenant une partie cylindrique pourvue sur sa périphérie externe d'une collerette circulaire adaptée pour former une butée d'appui du corps contre la paroi externe de l'os.

La description qui va suivre en regard des dessins annexés, donnés à titre d'exemples non limitatifs, permettra de mieux comprendre l'invention, les caractéristiques qu'elle présente et les avantages qu'elle est susceptible de procurer :
Figure 1 est une vue illustrant l'ancre de suture suivant la présente invention.
Figure 2 est une vue semblable à celle de figure 1, mais montrant une variante de l'ancre de suture suivant la présente invention.
Figure 3 est une vue représentant une autre variante de l'ancre de suture suivant la présente invention.
Figure 4a à 4d sont des vues schématiques illustrant la mise en place de l'ancre de suture suivant figure 1 et 2 dans un os pour la réinsertion des tissus mous.
Figure 5a à 5d sont des vues schématiques montrant la mise en place de l'ancre de suture suivant figure 3.
Figure 6a à 6d sont des vues schématiques représentant l'extraction de l'ancre de suture de l'os.

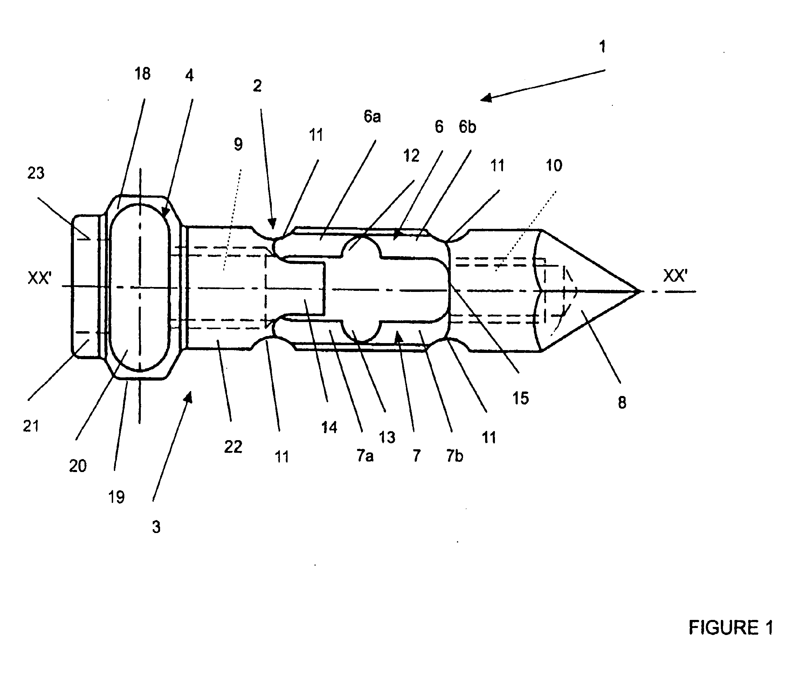

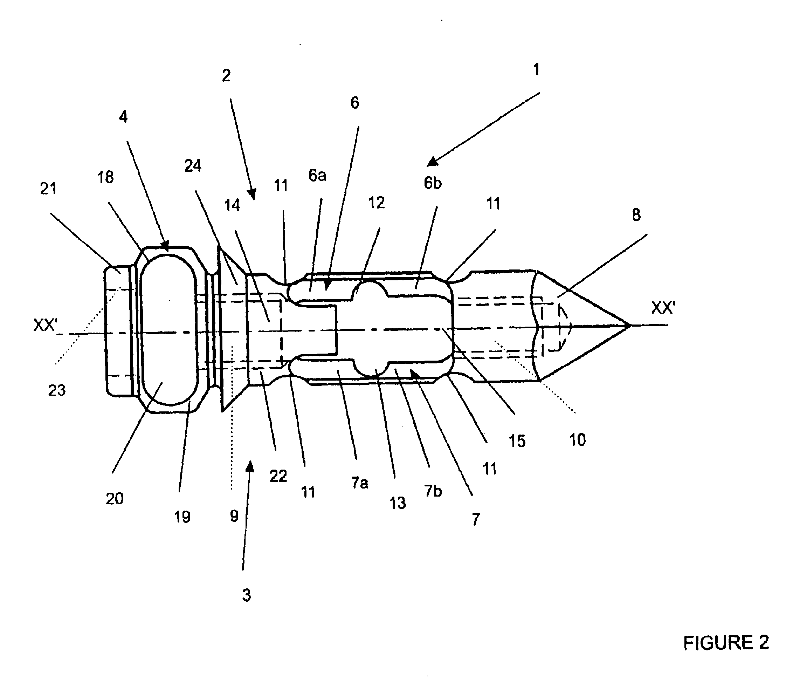

On a montré en figures 1 et 2 une ancre de suture monobloc 1 comportant un corps cylindrique creux 2 de forme allongée susceptible de se déformer plastiquement et pouvant être mis en place sur le site opératoire sous arthroscopie.

Le corps 2 est constitué à l'une de ses extrémités d'une tête 3 comportant des moyens de fixation 4 des tissus mous 16 contre l'os 17 d'un patient par l'intermédiaire d'un ou plusieurs fils de suture 5 sertis sur ladite tête.

La tête 3 est constituée des moyens de fixation 4 qui sont prévus entre une face cylindrique d'appui 21 et une partie cylindrique 22.

La partie cylindrique 22 de la tête 3 se prolonge à l'opposé des moyens de fixation 4 par au moins deux branches de fixation 6, 7 qui sont, avant déformation, parallèles à l'axe longitudinal XX' du corps 2.

L'autre extrémité du corps 2, se trouvant à l'opposé de celle de la tête 3, est constituée dans le prolongement des branches 6 et 7 d'une pointe à profil conique 8 facilitant la mise en place de l'ancre de suture dans l'os 17.

La partie cylindrique 22 de la tête 3 comporte un alésage interne fileté 9 qui débouche d'une part du coté des moyens de fixation 4 et d'autre part entre les branches de fixation 6 et 7. L'alésage fileté 9 est porté par l'axe longitudinal XX' du corps 2.

Également, la pointe à profil conique 8 présente dans sa partie interne un trou borgne fileté 10 qui débouche entre les branches de fixation 6 et 7 et qui est porté par le même axe longitudinal XX' que celui de l'alésage 9. En Outre, le diamètre de l'alésage fileté 9 est prévu plus grand que celui du trou fileté 10.

Les branches 6 et 7 sont raccordées à la tête 3 et à la pointe 8 par des amorces de pliage 11 dirigées en direction du centre du corps 2 et qui permettront de déformer lesdites branches sous un effort de traction.

Les branches 6 et 7 présentent respectivement en leur milieu une amorce de pliage 12, 13 qui est inversée par rapport à celle 11 de manière que chaque branche soit constituée de deux segments identiques 6a, 6b et 7a, 7b.

Entre chaque branche 6 et 7 sont prévus des moyens de butée 14 solidaires de la partie cylindrique 22 de la tête 3 et qui est dirigée en direction de la pointe à profil conique 8. Chaque butée 14 s'étend parallèlement à l'axe longitudinal XX' du corps 2 et présente une longueur qui dépend de la déformation que l'on désire obtenir des branches 6 et 7.

En effet, la déformation plastique des branches 6 et 7 est limitée par les butées 14 qui viennent en appui contre une face 15 de la pointe à profil conique 8. La face 15 est disposée dans un plan perpendiculaire à celui portant l'axe XX' du corps 2.

Les moyens de fixation 4 sont constitués de deux languettes 18, 19 opposées et parallèles à l'axe longitudinal XX' du corps 2, avant leur déformation. Les languettes 18 et 19 délimitent un espace oblong 20 qui permet le passage, par le chirurgien sur le site opératoire, d'au moins un fil de suture 5.

L'espace oblong 20 des moyens de fixation 4, est positionné de manière que sa direction de plus grande longueur soit perpendiculaire à l'axe longitudinal XX' du corps 2 de l'ancre de suture 1.

La face d'appui 21 réunissant les languettes 18, 19 à l'opposé de la partie cylindrique 22, est percée d'un alésage 23, coaxial à celui fileté 9, et qui débouche d'une part à l'extérieur du corps 2 et d'autre part entre lesdites languettes pour être positionné en face dudit alésage fileté 9 de ladite partie cylindrique 22.

La partie cylindrique 22 de la tête 3 comporte sur sa périphérie externe et entre les moyens de fixation 4 et les branches 6, 7 une collerette circulaire 24 formant une butée d'appui du corps 2 contre la paroi externe de l'os 17, lors de l'introduction de l'ancre de suture 1 à l'intérieur de ce dernier (figure 2). La collerette 24 présente un profil conique qui vient en contact avec l'os 17 pour servir de butée lors de l'effondrement des branches de fixation 6 et 7.

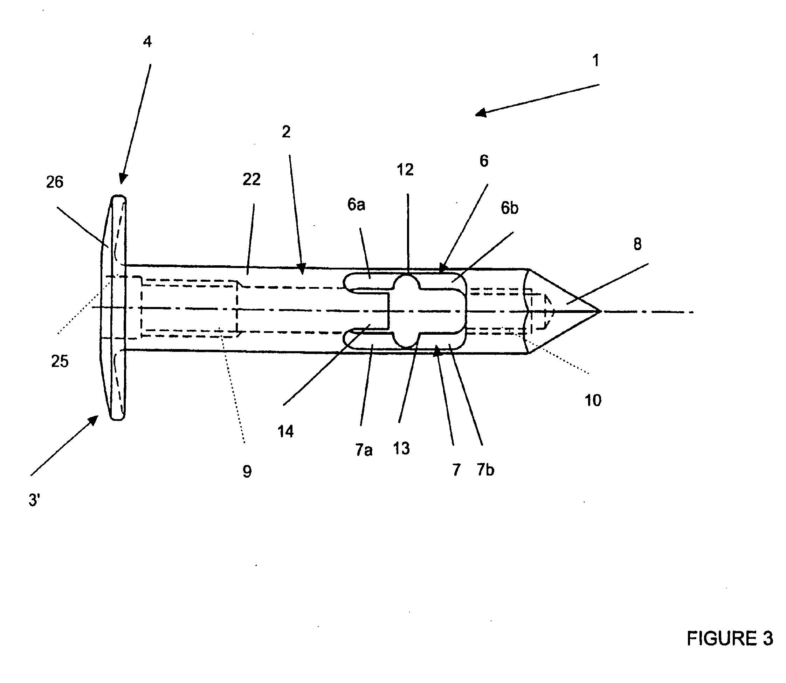

En figure 3 on a représenté une variante de l'ancre de suture 1 dont le corps cylindrique 2 présente une tête 3', différente de celle 3 décrite précédemment, en ce qui concerne les moyens de fixation 4 des tissus mous.

La tête 3' est constituée d'une partie cylindrique 22 plus longue que celle décrite précédemment et dans laquelle est percé l'alésage interne et fileté 9. Ce dernier débouche d'une part entre les branches 6, 7 et d'autre part à l'extérieur du corps 2 au niveau des moyens de fixation 4 par l'intermédiaire d'un alésage coaxial 25.

L'alésage interne 9 présente, sur sa longueur, une partie filetée qui est plus courte que celle montrée en figures 1 et 2. De plus, on remarque dans cette variante que la partie filetée de l'alésage interne 9 est éloignée des butées 14 pour se trouver à proximité des moyens de fixation 4, du fait de la partie cylindrique 22 plus longue.

Les moyens de fixation 4 sont constitués à l'extrémité de la partie cylindrique 22 de la tête 3' et à l'opposé des branches 6,7 par un disque 26 qui peut être conformé pour permettre la retenue des tissus mous 16 contre l'os 17.

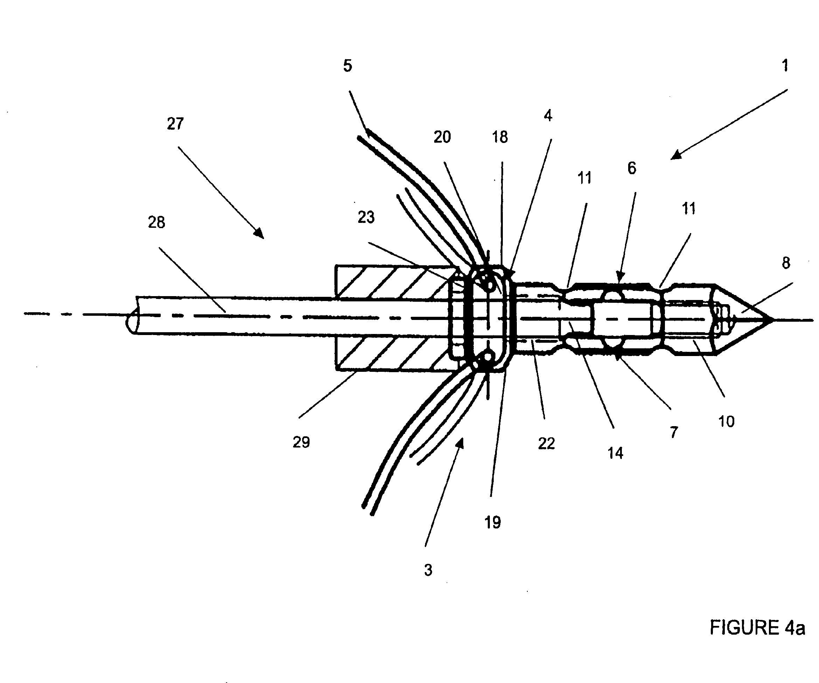

On a représenté en figures 4a à 4d les différentes étapes pour la mise en place de l'ancre de suture 1 décrite précédemment à l'intérieur de l'os 17 pour la fixation des tissus mous 16.

La figure 4a montre l'ancre de suture 1 munie de son ancillaire 27 de mise en place qui est constitué, par exemple, d'une tige 28 qui traverse le corps 2 pour venir se visser dans le trou borgne 10 de la pointe à profil conique 8. La tige 28 est solidaire d'un embout 29 qui vient prendre appui contre la face 23 de la tête 3.

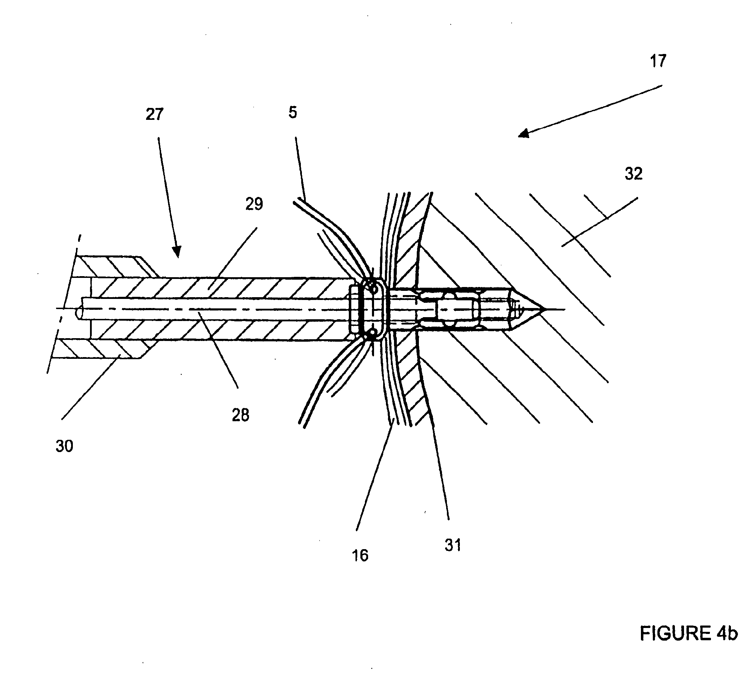

La figure 4b représente l'ancre de suture 1 qui est introduite dans le site opératoire par l'intermédiaire de l'ancillaire 27 et d'une canule d'arthroscopie 30. Le chirurgien procède à la mise en place des fils de suture 5 dans l'espace oblong 20 des moyens de fixation 4, soit avant l'introduction de l'ancre de suture 1 dans le site opératoire, soit après sa mise en place dans l'os 17.

La mise en place de l'ancre de suture 1 dans l'os 17 est réalisée soit par force, soit par rotation, soit par l'intermédiaire d'un pré-trou percé dans l'os cortical 31 et l'os spongieux 32, à travers le tissu mou 16 à réinsérer.

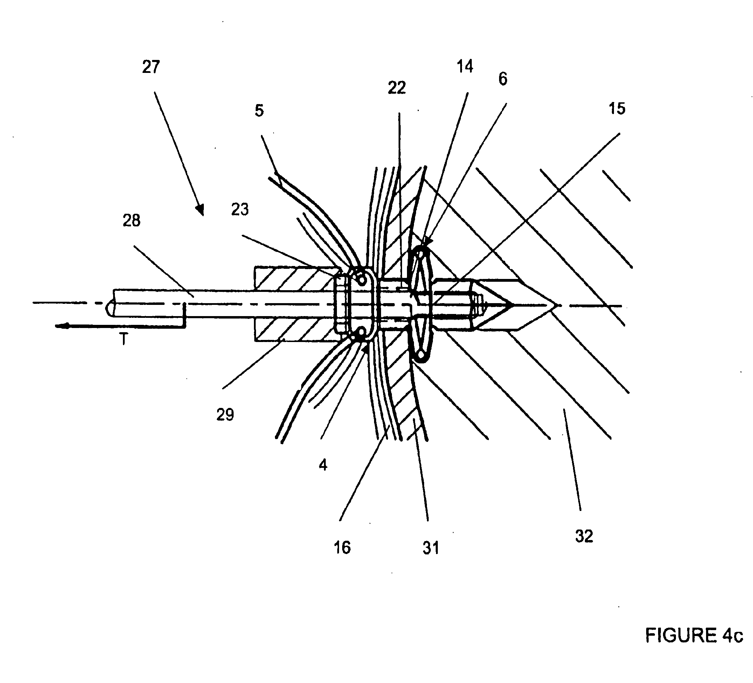

La figure 4c montre la déformation de l'ancre de suture 1 et plus particulièrement des branches 6 et 7 à l'intérieur de l'os spongieux 32 lorsqu'un effort de traction T est soumis à la tige 28 de l'ancillaire 27. Ainsi, la tige 28 se déplace horizontalement suivant l'axe XX' du corps 2, tandis que l'embout 29 reste fixe en appui contre la face 23 de la tête 3.

La déformation des branches 6 et 7 est limitée jusqu'à ce que la pointe à profil conique 8 vienne par l'intermédiaire de sa paroi 15 en appui contre les butées 14.

Les branches 6 et 7 se déforment, sous un effort de compression du fait de la traction T soumise à la tige 28 de l'ancillaire 27, suivant le profil des amorces 11, 12 et 13 de manière que les segments 6a, 6b et 7a, 7b soient dirigés à l'extérieur du corps 2 et dans une direction sensiblement perpendiculaire à l'axe XX'.

On note que la fixation de l'ancre de suture 1 dans l'os spongieux 32 est réalisée par la déformation des branches 6 et 7 jusqu'à ce que les segments 6a et 7a viennent en contact avec la face interne de l'os cortical 31.

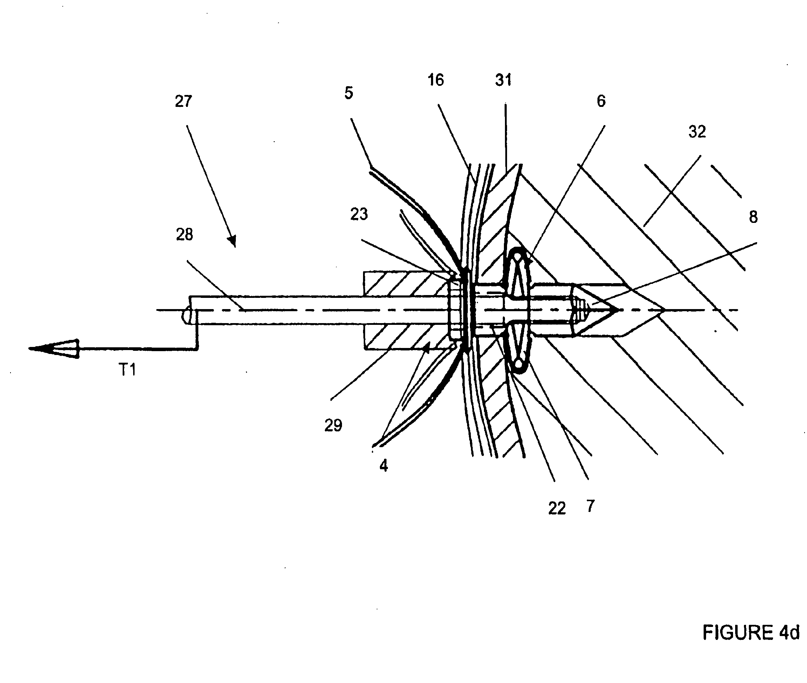

La figure 4d illustre le sertissage des fils de suture 5 sur la tête 3 de l'ancre 1 par l'intermédiaire des moyens de fixation 4.

La pointe à profil conique 8 étant en contact avec les butées 14, il est possible d'appliquer un effort de traction T1 plus important que celui T sur la tige 28 de l'ancillaire 27, sans risquer de rompre les branches 6 et 7, afin de déformer les languettes 18 et 19 des moyens de fixation 4.

La déformation des languettes 18 et 19 permet de réduire l'espace oblong 20 de manière à bloquer dans une position tendue les fils de suture 5 sur la tête 3 de l'ancre de suture 1.

Les languettes 18 et 19 sont conformées pour ne pas couper les fils de suture 5 lors du sertissage de ces derniers sur la tête 3.

Les fils de suture 5 permettent aux chirurgiens de ligaturer les tissus mous 16 sur l'ancre de suture 1.

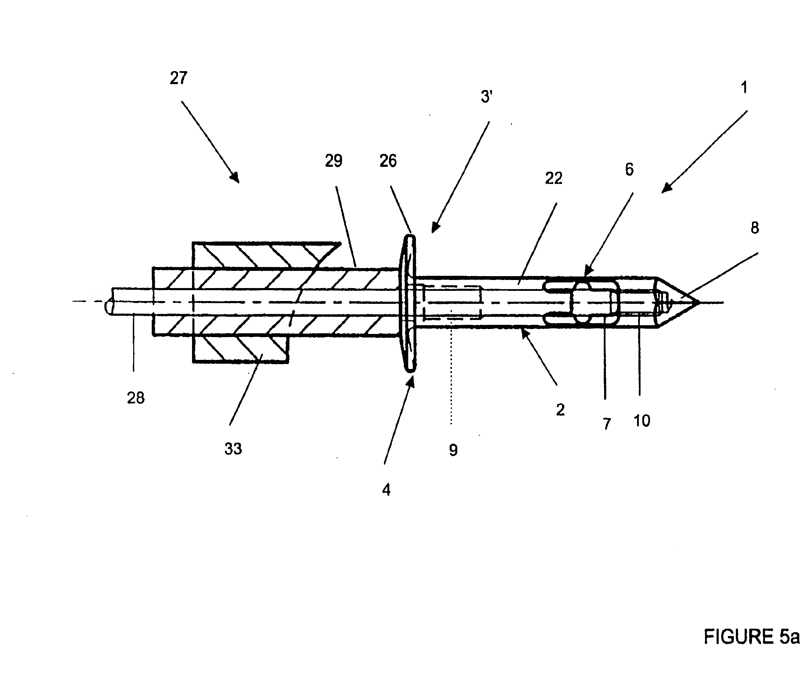

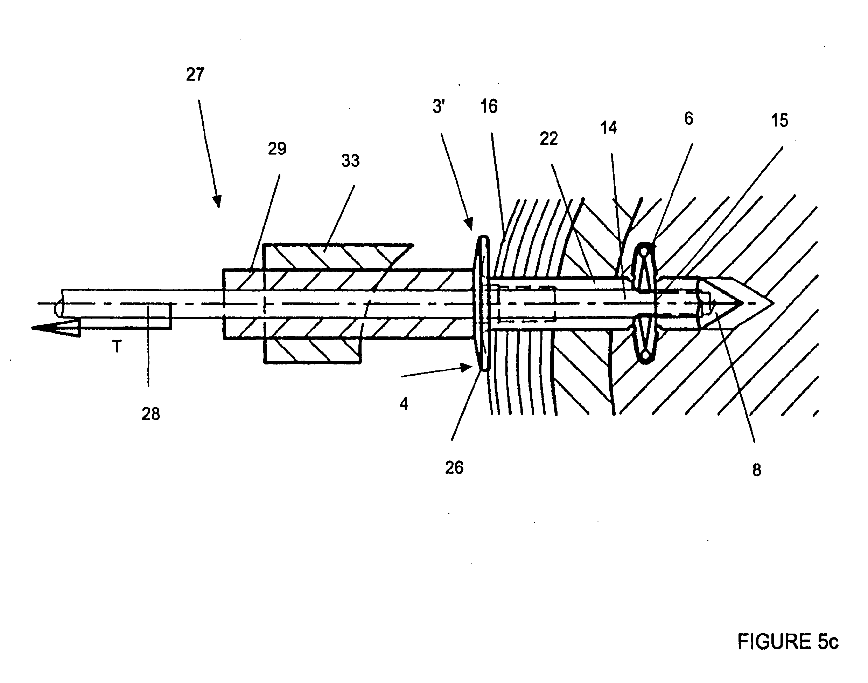

On a représenté en figures 5a à 5d les différentes étapes pour la mise en place de l'ancre de suture 1 décrite précédemment en figure 3 à l'intérieur de l'os 17 pour la fixation des tissus mous 16.

La figure 5a montre l'ancre de suture 1 munie de son ancillaire 27 de mise en place qui est constitué de la tige 28 qui traverse le corps 2 pour venir se visser dans le trou borgne 10 de la pointe à profil conique 8. La tige 28 est solidaire d'un embout 29 qui vient prendre appui contre le disque 26 de la tête 3'.

Autour de l'embout 29 est prévu un outil de mise en forme 33 du disque 26 sur le tissu mou 16.

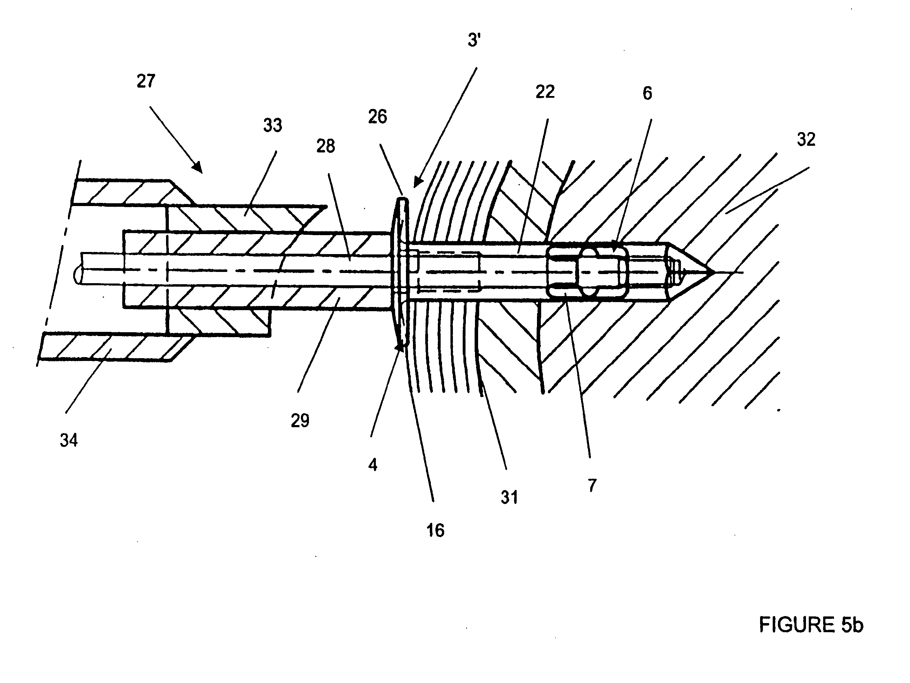

La figure 5b représente l'ancre de suture 1 qui est introduite dans le site opératoire par l'intermédiaire de l'ancillaire 27 et d'une canule d'arthroscopie 34.

La mise en place de l'ancre de suture 1 dans l'os 17 est réalisée soit par force, soit par rotation, soit par l'intermédiaire d'un pré-trou percé dans l'os cortical 31 et l'os spongieux 32, à travers le tissu mou 16 à réinsérer.

La figure 5c montre la déformation de l'ancre de suture 1 et plus particulièrement des branches 6 et 7 à l'intérieur de l'os spongieux 32 lorsqu'un effort de traction T est soumis à la tige 28 de l'ancillaire 27. Ainsi, la tige 28 se déplace horizontalement suivant l'axe XX' du corps 2, tandis que l'embout 29 reste fixe en appui contre le disque 26 de la tête 3'.

La déformation des branches 6 et 7 est limitée jusqu'à ce que la pointe à profil conique 8 vienne par l'intermédiaire de sa face 15 en appui contre les butées 14.

Les branches 6 et 7 se déforment, sous un effort de compression du fait de la traction T soumise à la tige 28 de l'ancillaire 27, suivant le profil des amorces 11, 12 et 13 de manière que les segments 6a, 6b et 7a, 7b soient dirigés à l'extérieur du corps 2 et dans une direction sensiblement perpendiculaire à l'axe XX'.

On note que la fixation de l'ancre de suture 1 dans l'os spongieux 32 est réalisée par la déformation des branches 6 et 7 jusqu'à ce que les segments 6a et 7a viennent en contact avec la face interne de l'os cortical 31.

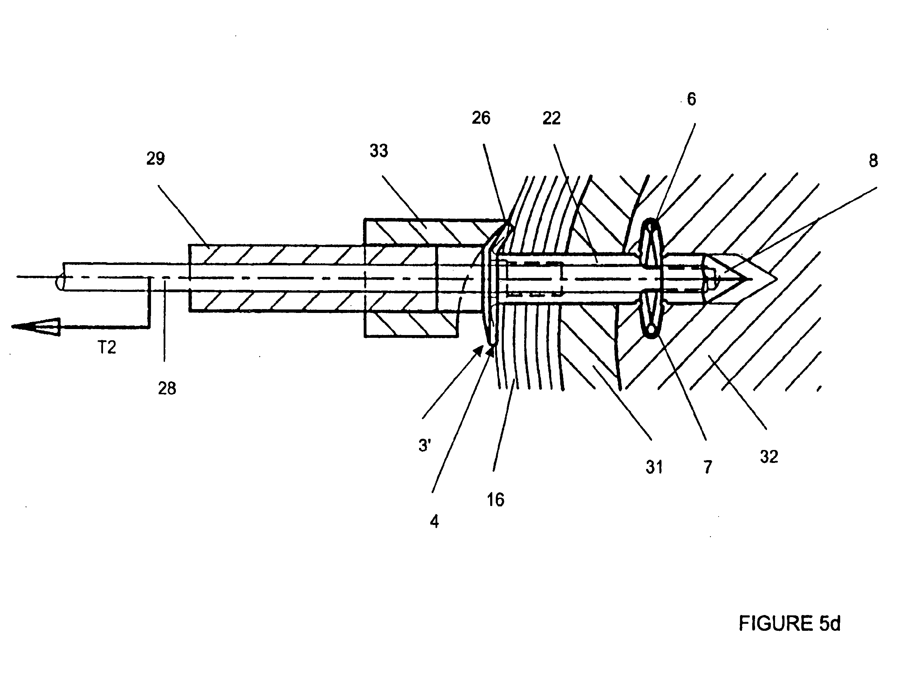

En figure 5d on a montré la conformation du disque 26 par l'outil 33, après retrait préalable de l'embout 29, afin d'appliquer un nouvel effort de traction T2 supérieur à celui T permettant la déformation des branches 6 et 7.

L'effort T2 permet au chirurgien de plier le disque 26 de la tête 3' pour qu'il pénètre dans le tissu mou 16 afin de le plaquer contre l'os cortical 31.

La conformation ou le pliage du disque 26 est réalisé en plusieurs fois. Il suffit au chirurgien de dévisser la tige 28 pour positionner l'outil 33 suivant une autre direction, de revisser la tige 28 dans le trou 10 de la pointe 8 et d'appliquer de nouveau l'effort de traction T2 pour plier le disque 26.

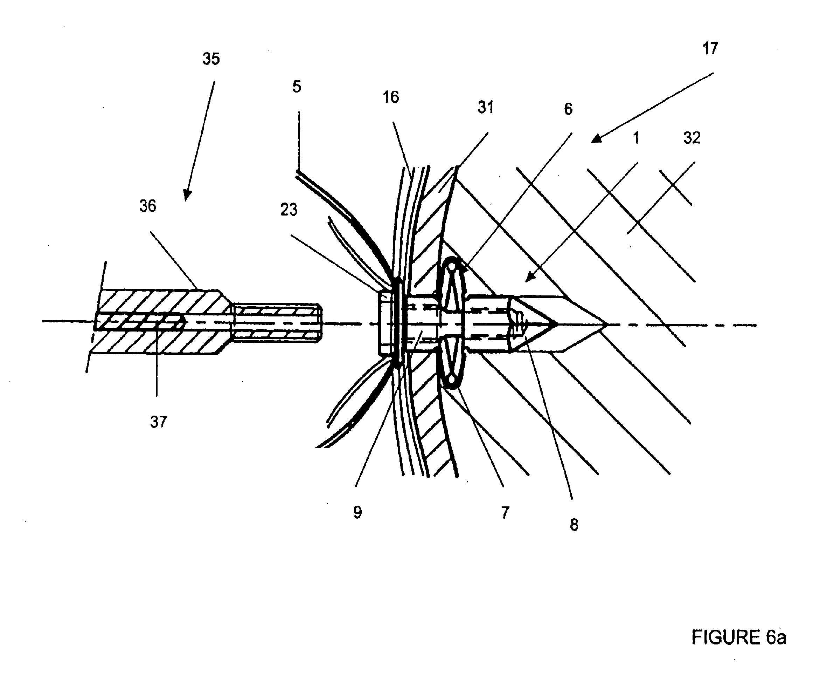

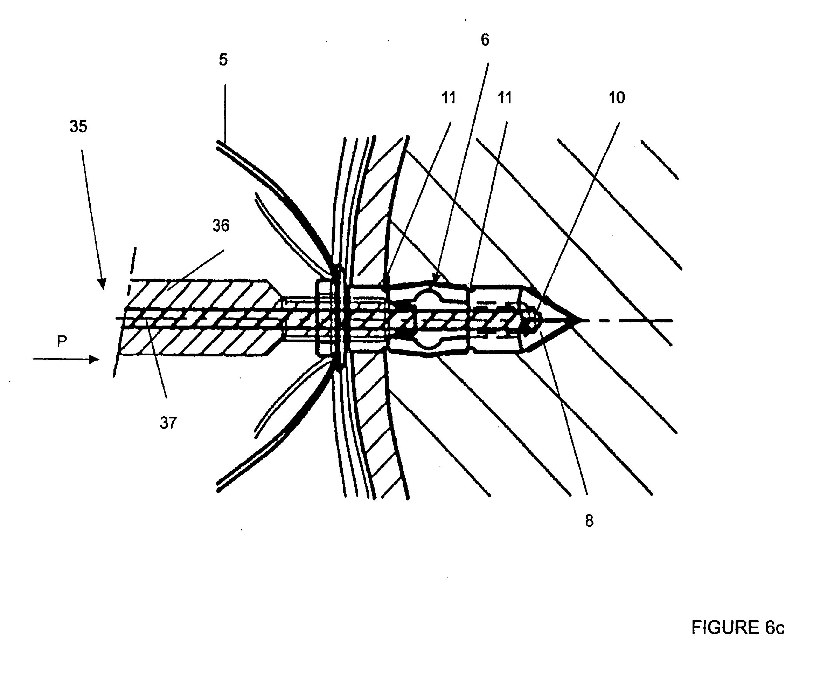

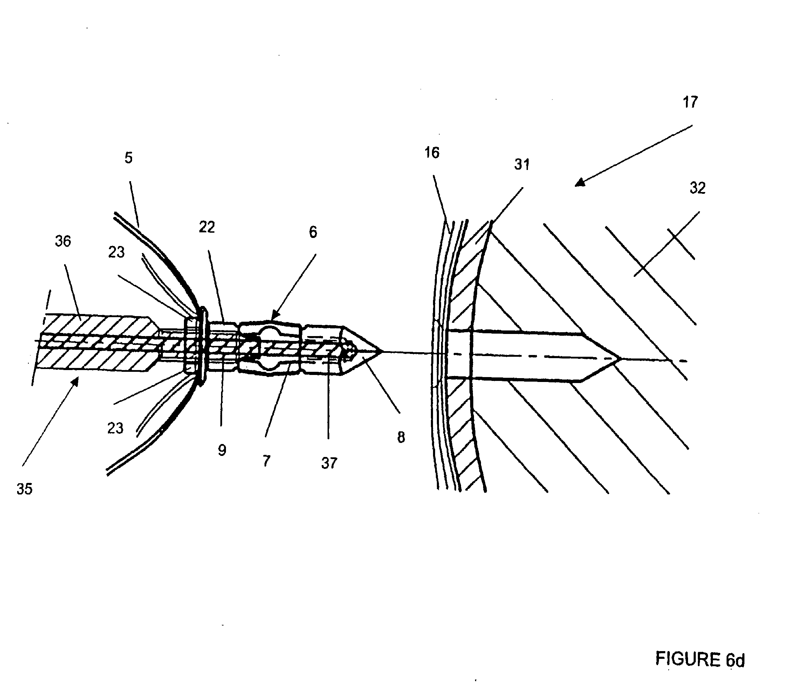

En figures 6a à 6d on a illustré les différentes étapes pour extraire l'ancre de suture 1 de l'os 17 au moyen d'un autre ancillaire 35. Ce dernier permet l'extraction de l'ancre de suture 1 comportant la tête 3 ou 3'.

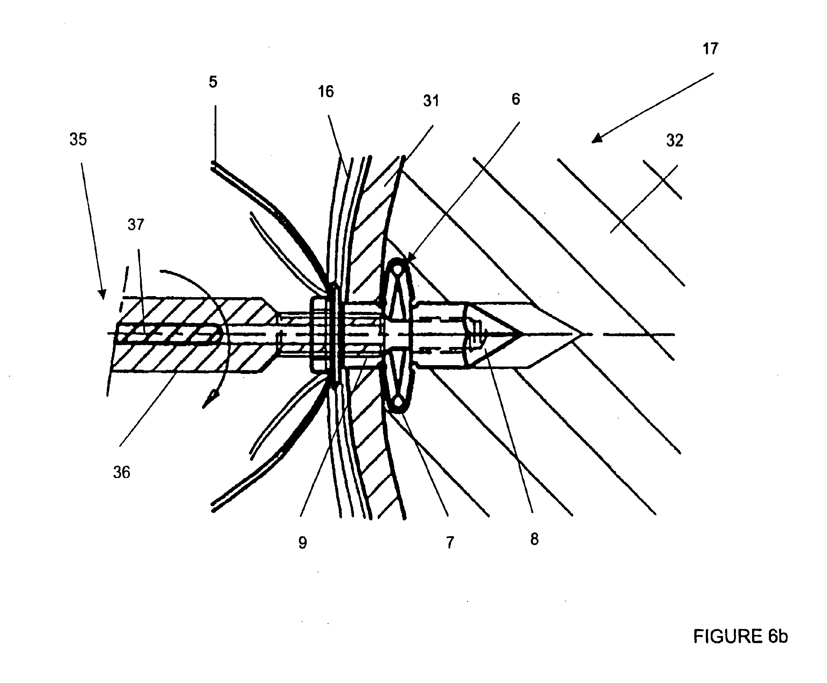

L'ancillaire 35 comporte une tige creuse 36 qui vient se visser dans l'alésage fileté 9 de la partie cylindrique 22, tandis qu'une autre tige 37 coulissant dans la première vient prendre appui dans le fond du trou borgne 10 ménagé dans la pointe à profil conique 8 (figure 6b).

La tige 36 est soumise à un effort de poussée P parallèle à l'axe XX' du corps 2, afin de déplier les branches 6 et 7 (figure 6c). Le profil des amorces 11, 12 et 13 permet de ramener l'ancre de suture 1 suivant une forme semblable à celle d'origine.

Dès que l'ancre de suture 1 a retrouvé une position allongée, le chirurgien peut, à l'aide de l'ancillaire 35, retirer ladite ancre de l'os 17, sans avoir à percer un trou dont le diamètre est sensiblement voisin de celui affecté par les branches déformées.

## Revendications

1. Ancre de suture permettant la fixation de tissus mous (16) contre un os (17), comportant un corps creux (2) comprenant à l'une de ses extrémité une tête (3) pourvue de moyens de fixation (4) des tissus mous (16) contre l'os (17) par le passage d'au moins un fil de suture (5), **caractérisée en ce que** les moyens de fixation (4) sont constitués de deux languettes (18, 19) opposées et parallèles à l'axe longitudinal (XX') du corps creux (2), avant déformation, afin de délimiter un espace oblong (20) qui est traversé par au moins un fil de suture (5), ledit fil de suture (5) étant serti sur la tête (3) après déformation des languettes (18, 19) sous un effort de traction pour permettre la ligature des tissus mous (16) sur l'ancre de suture (1).

2. Ancre de suture suivant la revendication 1, **caractérisée en ce qu**'elle comporte un corps creux (2) comprenant des moyens d'expansion réversibles qui sont constitués par au moins deux branches de fixation (6, 7) disposées dans le prolongement de ladite tête (3) et parallèlement à l'axe longitudinal (XX') dudit corps avant déformation, au moins deux butées (14) intercalées entre chaque branche de fixation (6, 7) et limitant la déformation plastique de ces dernières lors de l'application d'un effort extérieur de traction (T) pour la fixation de l'ancre de suture (1) dans l'os (17) afin que la déformation desdits moyens d'expansion (6, 7) soit réversible lors de l'application d'un autre effort extérieur de poussée (P) permettant le retrait de ladite ancre de suture (1) de l'os (17).

3. Ancre de suture suivant l'une quelconque des revendications 1 et 2, **caractérisée en ce que** le corps creux (2) comporte une pointe à profil conique (8) dans laquelle est ménagé un trou borgne fileté (10) qui est prévu pour recevoir une tige filetée (28) d'un ancillaire (27) pour déformer, d'une part sous un premier effort de traction (T) les branches de fixation (6, 7) suivant une direction sensiblement perpendiculaire à l'axe longitudinal (XX') dudit corps et d'autre part sous un second effort de traction (T1, T2) supérieur au premier (T) les moyens de fixation (4).

4. Ancre de suture suivant la revendication 3, **caractérisée en ce que** le corps creux (2) comporte à proximité de la tête (3) un alésage interne fileté (9) qui est destiné à recevoir une première tige filetée creuse (36) d'un ancillaire (35), tandis qu'une seconde tige (37) coulissant dans la première tige (36) vient en appui dans le fond d'un trou borgne fileté (10) pour déplier sous un effort de poussée (P) les branches de fixation (6, 7) dans une position sensiblement allongée pour pouvoir extraire l'ancre de suture (1) de l'os (17).

5. Ancre de suture suivant la revendication 2, **caractérisée en ce que** les branches de fixation (6, 7) sont raccordées à une partie cylindrique (22) de la tête (3) et à la pointe à profil conique (8) par des premières amorces de pliage (11) dirigées en direction du centre du corps (2).

6. Ancre de suture suivant la revendication 5, **caractérisée en ce que** les branches de fixation (6, 7) présentent respectivement en leur milieu une seconde amorce de pliage (12, 13) qui est inversée par rapport aux premières amorces de pliage (11) de manière que chaque branche soit constituée de deux segments identiques (6a, 6b ; 7a, 7b).

7. Ancre de suture suivant la revendication 4, **caractérisée en ce que** l'alésage interne fileté (9) et le trou borgne fileté (10) sont portés par le même axe longitudinal (XX') du corps (2) et sont prévus de diamètres différents.

8. Ancre de suture suivant la revendication 2, **caractérisée en ce que** les butées (14) s'étendent parallèlement à l'axe longitudinal (XX') du corps (2) et présentent une longueur qui dépend de la déformation que l'on désire obtenir des branches de fixation (6, 7).

9. Ancre de suture suivant la revendication 3, **caractérisée en ce que** l'espace oblong (20) des moyens de fixation (4) est positionné de manière que sa direction de plus grande longueur soit perpendiculaire à l'axe longitudinal (XX') du corps (2).

10. Ancre de suture suivant la revendication 3, **caractérisée en ce que** la tête (3) comporte une partie cylindrique (22) pourvue sur sa périphérie externe d'une collerette circulaire (24) adaptée pour former une butée d'appui du corps (2) contre la paroi externe de l'os (17).

## Claims

1. Suture anchor permitting the fixing of soft tissues (16) against a bone (17), comprising a hollow body (2) comprising at one of its ends a head (3) provided with means (4) for fixing soft tissues (16) against bone (17) by way of the transit of at least one suture thread (5), **characterised in that** the fixing means (4) are constituted by two strips (18, 19) opposite and parallel to the longitudinal axis (XX') of the hollow body (2) prior to bending in order to delimit an oblong space (20), which is traversed by at least one suture thread (5), said suture thread (5) being set on the head (3) following bending of the strips (18, 19) subject to a pulling force in order to permit the ligature of the soft tissues (16) on the suture anchor (1).

2. Suture anchor according to claim 1, **characterised in that** it comprises a hollow body (2) comprising reversible expansions means, which are constituted by at least two fixing arms (6, 7) arranged in the extension of said head (3) and in parallel to the longitudinal axis (XX') of said body prior to bending, at least two stops (14) inserted between each fixing arm (6, 7) and limiting the plastic bending of the latter during the application of an external pulling force (T) in order to fix the suture anchor (1) in the bone (17) in order that the bending of said expansion means (6, 7) be reversible during the application of another external pushing force (P), which permits the withdrawal of said suture anchor (1) from the bone (17).

3. Suture anchor according to any one of claims 1 and 2, **characterised in that** the hollow body (2) comprise a point with a conical profile (8), in which a blind threaded hole (10) is arranged, which is provided in order to receive a threaded rod (28) of a jig (27) for bending the fixing arms (6, 7) on the one hand subject to a first pulling force (T) in a direction approximately perpendicular to the longitudinal axis (XX') of said body and on the hand the fixing means (4) subject to a second pulling force (T1, T2) in excess of the first pulling force (T).

4. Suture anchor according to claim 3, **characterised in that** the hollow body (2) comprises an internal threaded bore (9) near the head (3), which is intended for receiving a first threaded hollow rod (36) of a jig (35), whereas a second rod (37) sliding in the first rod (36) engages in the bottom of a blind threaded hole (10) for opening out the fixing arms (6, 7) subject to a pushing force (P) in a approximately elongated position in order to be able to remove the suture anchor (1) from the bone (17).

5. Suture anchor according to claim 2, **characterised in that** the fixing arms (6, 7) are connected to a cylindrical part (22) of the head (3) and to the point with a conical profile (8) by the initial bending areas (11) directed in the direction of the centre of the body (2).

6. Suture anchor according to claim 5, **characterised in that** the fixing arms (6, 7) respectively have in their middle a second initial bending area (12, 13), which is reversed with reference to the first initial bending areas (11) in such a way that each arm is constituted by two identical segments (6a, 6b; 7a, 7b).

7. Suture anchor according to claim 4, **characterised in that** the internal threaded bore (9) and the blind threaded hole (10) are carried by the same longitudinal axis (XX') of the body (2) and are provided with different diameters.

8. Suture anchor according to claim 2, **characterised in that** the stops (14) extend in parallel along the longitudinal axis (XX') of the body (2) and have a length depending on the bending that one wishes to obtain for the fixing arms (6, 7).

9. Suture anchor according to claim 3, **characterised in that** the oblong space (20) of the fixing means (4) is positioned in such a way that its longest direction is perpendicular to the longitudinal axis (XX') of the body (2).

10. Suture anchor according to claim 3, **characterised in that** the head (3) comprises a cylindrical part (22), which is provided on its external periphery with a circular flange (24) adapted to form a stop for engaging the body (2) against the external wall of the bone (17).

## Patentansprüche

1. Nahtanker, der die Befestigung von Weichgeweben (16) an einem Knochen (17) ermöglicht, umfassend einen Hohlkörper (2), der an einem seiner Enden einen Kopf (3) aufweist, der mit Befestigungsmitteln (4) der Weichgewebe (16) am Knochen (17) durch Durchführen von mindestens einem Nähfaden (5) versehen ist, **dadurch gekennzeichnet, dass** die Befestigungsmittel (4) von zwei Zungen (18, 19) gebildet sind, die gegenüber liegend und parallel zur Längsachse (XX') des Hohlkörpers (2) vor der Verformung angeordnet sind, um einen länglichen Raum (20) zu begrenzen, der von mindestens einem Nähfaden (5) durchquert wird, wobei der Nähfaden (5) auf dem Kopf (3) nach Verformung der Zungen (18, 19) unter einer Zugkraft gezogen wird, um die Ligatur der Weichgewebe (16) mit dem Nahtanker (1) zu ermöglichen.

2. Nahtanker nach Anspruch 1, **dadurch gekennzeichnet, dass** er einen Hohlkörper (2) aufweist, umfassend reversible Spreizmittel, die von mindestens zwei Befestigungsabschnitten (6, 7) gebildet sind, die in der Verlängerung des Kopfes (3) und parallel zur Längsachse (XX') des Körpers vor der Verformung angeordnet sind, mindestens zwei Anschläge (14), die zwischen jedem Befestigungsabschnitt (6, 7) angeordnet sind und die plastische Verformung dieser letztgenannten beim Anlegen einer äußeren Zugkraft (T) zur Befestigung des Nahtankers (1) im Knochen (17) begrenzen, so dass die Verformung der Spreizmittel (6, 7) beim Anlegen einer weiteren äußeren Schubkraft (P), die das Herausziehen des Nahtankers (1) aus dem Knochen (17) ermöglicht, reversibel ist.

3. Nahtanker nach einem der Ansprüche 1 und 2, **dadurch gekennzeichnet, dass** der Hohlkörper (2) eine Spitze mit konischem Profil (8) umfasst, in der ein Grundloch mit Gewinde (10) angeordnet ist, das dazu vorgesehen ist, eine Gewindestange (28) eines Hilfsgeräts (27) aufzunehmen, um einerseits unter einer ersten Zugkraft (T) die Befestigungsabschnitte (6, 7) in eine im Wesentlichen auf die Längsachse (XX') des Körpers senkrechte Richtung und andererseits unter einer zweiten Zugkraft (T1, T2), die größer als die erste (T) ist, die Befestigungsmittel (4) zu verformen.

4. Nahtanker nach Anspruch 3, **dadurch gekennzeichnet, dass** der Hohlkörper (2) in der Nähe des Kopfes (3) eine innere Gewindebohrung (9) umfasst, die dazu bestimmt ist, eine erste hohle Gewindestange (36) eines Hilfsgeräts (35) aufzunehmen, während eine zweite Stange (37), die in der ersten Stange (36) gleitet, am Boden eines Grundlochs mit Gewinde (10) zur Anlage gelangt, um unter einer Schubkraft (P) die Befestigungsabschnitte (6, 7) in eine im Wesentlichen längliche Position aufzubiegen, um den Nahtanker (1) aus dem Knochen (17) herauszuziehen.

5. Nahtanker nach Anspruch 2, **dadurch gekennzeichnet, dass** die Befestigungsabschnitte (6, 7) an einen zylindrischen Teil (22) des Kopfes (3) und an die Spitze mit konischem Profil (8) durch erste Biegeansätze (11), die in Richtung der Mitte des Körpers (2) gerichtet sind, angeschlossen sind.

6. Nahtanker nach Anspruch 5, **dadurch gekennzeichnet, dass** die Befestigungsabschnitte (6, 7) in ihrer Mitte jeweils einen zweiten Biegeansatz (12, 13) aufweisen, der in Bezug auf die ersten Biegeansätze (11) umgekehrt ist, damit jeder Abschnitt von zwei identischen Segmenten (6a, 6b; 7a, 7b) gebildet ist.

7. Nahtanker nach Anspruch 4, **dadurch gekennzeichnet, dass** die innere Gewindebohrung (9) und das Grundloch mit Gewinde (10) auf derselben Längsachse (XX') des Körpers (2) liegen und mit unterschiedlichen Durchmessern versehen sind.

8. Nahtanker nach Anspruch 2, **dadurch gekennzeichnet, dass** sich die Anschläge (14) parallel zur Längsachse (XX') des Körpers (2) erstrecken und eine Länge aufweisen, die von der Verformung abhängt, die von den Befestigungsabschnitten (6, 7) zu erhalten gewünscht wird.

9. Nahtanker nach Anspruch 3, **dadurch gekennzeichnet, dass** der längliche Raum (20) der Befestigungsmittel (4) derart angeordnet ist, dass seine Richtung senkrecht zur Längsachse (XX') des Körpers (2) von größerer Länge ist.

10. Nahtanker nach Anspruch 3, **dadurch gekennzeichnet, dass** der Kopf (3) einen zylindrischen Teil (22) umfasst, der an seiner äußeren Peripherie mit einem kreisförmigen Kragen (24) versehen ist, der dazu ausgebildet ist, einen Stützanschlag für den Körper (2) an der Außenwand des Knochens (17) zu bilden.
